# EUROPEAN PATENT SPECIFICATION

(11) **EP 3 194 508 B1**
(45) Date of publication and mention of the grant of the patent: **21.10.2020**
(21) Application number: 15791783.2
(22) Date of filing: 16.09.2015
(51) Int. Cl.: C09D 5/00

(54) **METHOD FOR PROVIDING A SUBSTRATE WITH AN ANTIMICROBIAL COATING, AND COATED SUBSTRATES OBTAINABLE THEREBY**
VERFAHREN ZUM VERSEHEN EINES SUBSTRATS MIT EINER ANTIBAKTERIELLEN BESCHICHTUNG UND BESCHICHTETES SUBSTRAT DARAUS
PROCÉDÉ PERMETTANT D'OBTENIR UN SUBSTRAT AVEC UN REVÊTEMENT ANTIBACTÉRIEN ET SUBSTRATS REVÊTUS OBTENUS PAR CES PROCÉDÉS

(30) Priority: 16.09.2014 EP 14184939
(43) Date of publication of application: 26.07.2017
(73) Proprietor: Zorg Innovaties Nederland B.V., 1054 ET Amsterdam (NL)
(72) Inventor: LOONTJENS, Jacobus Antonius, NL-9747 AG Groningen (NL); BUSSCHER, Hendrik Jan, NL-9713 AV Groningen (NL); VAN DER MEI, Henderina Catharina, NL-9713 AV Groningen (NL); MUSZANSKA, Agnieszka Karolina, NL-1054 ET Amsterdam (NL); ROEST, Steven, NL-9713 AV Groningen (NL)
(74) Representative: V.O.
(86) International application number: PCT/NL2015/050637
(87) International publication number: WO 2016/043584

(56) References cited:
- WO-A1-2012/053655
- US-A- 5 908 808

## Description

The invention relates to the field of antimicrobial materials, in particular to implantable and other medical devices, exhibiting antimicrobial activity. More specifically, the present invention is directed to a method for providing a substrate, e.g. a catheter or another medical device or implant, with an antimicrobial coating comprising surface-immobilized quaternary ammonium compounds, and coated substrates obtainable thereby. It also relates to methods for reducing microbial biofilm formation on the surface of a substrate and to methods for reducing or eliminating the incidence of an implant-associated infection.

Infections associated with medical implants represent a major health care problem. For example, 5% of patients admitted to an acute care facility develop a hospital acquired infection. Hospital acquired infections (nosocomial infections) are the 11th leading cause of death in the US and cost over $2 billion annually. Nosocomial infections directly cause 19,000 deaths per year in the US and contribute to over 58,000 others.

The four most common causes of nosocomial infections are: urinary tract infection (28%); surgical site infection (19%); respiratory tract infection (17%); and bloodstream infection (16% and rising). A significant percentage of these infections are related to bacterial colonization of implanted medical implants such as Foley catheters (urinary tract infections), endotracheal and tracheostomy tubes (respiratory tract infections), and vascular infusion catheters (bloodstream infections). Although any infectious agent can infect medical implants, Gram positive bacteria such as Staphylococci (*S. aureus, S. epidermidis, S. pyogenes*), and Gram negative bacteria such as *Escherichia coli* (*E. coli*) and *Pseudomonas aeruginosa* are common pathogens. Once a medical implant becomes colonized by bacteria, it must frequently be replaced resulting in increased morbidity for the patient and increased cost to the healthcare system. Often the infected device serves as a source for a disseminated infection, which can lead to significant morbidity or even death.

Bacteria have a natural ability to attach themselves to surfaces, both natural and synthetic. Once attached, they often work cooperatively to form biofilms, thin layers of bacterial colonies that can cover the surface of a medical device and introduce the risk of infection. Due to their ability to produce a protective polysaccharide layer they become resistant against the immune system and administered antibiotics. As a result, orthopedic implants, catheters, and even contact lenses can become vehicles for infection.

The time course of infection development is not fully understood and varies greatly. However, it has been reported that approximately thirty percent of infections arise less than one month post-implant, another thirty-five percent occur between one month and twelve months post-implant, and the remainder appear more than a year post-implant.

Existing approaches against microbial adhesion use the release of antibiotics or silver from a coated surface of an implant. However, overuse of antibiotics can lead to bacterial resistance and the silver coating can be toxic to blood, limiting the clinical implementation of these methods. Moreover, releasing system are exhausted within a short period.

Quaternary-ammonium-compounds (QACs) are potent cationic antimicrobials used in everyday consumer products. Surface-immobilized, quaternary-ammonium-compounds create an antimicrobial contact-killing coating. Antimicrobial efficacy of QACs remains preserved when QAC-molecules are immobilized on a surface. Such contact-killing coatings have potential in widely-varying applications, including but not limited to surgical equipment and protective apparel in hospitals, medical implants and wound dressings, water purification, food packaging materials and industrial equipment. Use of QACs has been popular since they are easily produced and can be conveniently incorporated in coating systems. QACs are also stable in the human body, poorly metabolized and mainly excreted in nonmetabolized form. However, QACs can be hemolytic when not immobilized on a surface and environmentally toxic.

Behlau et al. (Biomaterials. Dec 2011; 32(34): 8783-8796) describe the biocompatibility and antibacterial properties of N,N-hexyl,methyl-polyethylenimine (HMPEI) covalently attached to the Boston Keratoprosthesis materials. It was found that HMPEI-derivatized materials exert an inhibitory effect on biofilm formation by *Staphylococcus aureus* clinical isolates, as compared to the parent poly(methyl methacrylate) (PMMA) and titanium.

Park et al. (Biotechnol. Prog. 2006, 22, 584-589) developed hydrophobic polycationic systems based on QACs that can serve as such a "bactericidal paint". Disclosed is a single-step, general procedure akin to common painting. Glass or polyethylene slides were briefly dipped into organic solutions of certain optimally hydrophobic N-alkyl-PEI (where PEI stands for branched 750-kDa polyethylenimine) polycations, followed by solvent evaporation. The N-alkyl-PEI was physically absorbed to the surface. The resultant polycation coated slides were able to kill on contact all of the encountered bacterial cells, whether the Gram-positive human pathogen *Staphylococcus aureus* or its Gram-negative brethren *Escherichia coli.* See also US2013/0110237.

However, QACs immobilized on a surface only exert contact-killing when the positive charge density is above a well-defined threshold. Accordingly, a limitation of typical contact-killing coatings is that the contact area between an adhering organism and a coating is generally small and mainly depending on the deformability of the rigid bacterial cell surface.

Asri et al. (Adv. Funct. Mater. 2013) addressed this issue by developing a shape-adaptive, multi-layered coating on which multiple QAC-molecules can be covalently tethered which is able to kill bacteria by partially enveloping them. Briefly, the method comprises covalently grafting onto silanol groups of a glass surface a specific coupling agent comprising blocked isocyanate groups, covalently anchoring to the coupling agent a polyurea coating by allowing polymerization of a monomer on the surface, and providing the hyperbranched coating with QACs.

Even after extensive washing, coatings thus obtained caused high contact-killing of *Staphylococcus epidermidis,* both in culture-based assays and through confocal-laser-scanning-microscopic examination of the membrane-damage of adhering bacteria. Staphylococcal adhesion forces to hyperbranched quaternary-ammonium coatings were extremely high, indicating that quaternary-ammonium-molecules on hyperbranched polyurea partially envelope adhering bacteria upon contact. However, whereas the potential of this shape-adaptive, flexible contact-killing coating for use in clinical applications is clear, the present inventors found that the glass coating procedure taught by Asri *et al.* did not yield satisfactory results when applied to materials that are typically used for medical implants, like titanium or medical grade polymers such as polydimethylsiloxane elastomer (PDMS). More in particular, adherence of the hyperbranched coating was found to be uneven and highly variable, with a highly irregular covering of the surface. They therefore set out to adapt the coating method such that it allows for providing medical implants that inherently resist microbial (e.g. bacterial) infection long-term.

It was surprisingly found that this goal can be achieved by allowing 'pre-oligomerization" of the monomers forming the hyperbranched polyurea to a low molecular weight polyurea prior to contacting the polyurea with the surface to be coated, rather than polymerization of the monomers on the surface. The coating obtained via pre-oligomerization was robust, reproducible and long-lasting, with a homogenous covering of the surface. The coating could be readily provided with antibacterial agents, including QACs.

Accordingly, the invention provides a method for providing a substrate with an antimicrobial coating by immobilizing a quaternary ammonium compound onto the surface of said substrate, comprising the steps of : (i) providing a surface comprising reactive hydroxyl groups; (ii) covalently grafting onto said reactive hydroxyl groups a siloxane coupling agent comprising a blocked isocyanate group;
(iii) polycondensation of AB₂ monomers comprising a secondary amine as A-group and blocked isocyanates as B-groups to a low number average molecular weight (Mₙ) polyurea of at least 2500 Da, preferably at least 4000 Da; (iv) contacting said low molecular weight polyurea to the surface grafted with coupling agent to covalently anchor the polyurea, and continuing polycondensation by heating the surface, optionally in the presence of AB₂ monomers, to obtain a hyperbranched polyurea coating; followed by (v) immobilizing onto said hyperbranched polyurea coating a hydrophobic N-alkylated polyethylenimine (PEI) having antimicrobial properties.

A method provided herein is not taught or suggested in the prior art.

US5,908,808 relates to coating formulations useful for the formation of a back layer on heat-sensitive recording materials such as wax-type thermal transfer materials or sublimation-type thermal transfer materials. Disclosed is a back-side coating formulation comprising, in a liquid medium, a reaction product obtained by reacting (a) a polysiloxane compound containing at least one functional group reactive with an isocyanate group, (b) a silane coupling agent containing at least one functional group selected from a functional group reactive with an isocyanate group or an isocyanate group, and (c) a polyisocyanate. US5,908,808 is completely silent about antimicrobial coatings, and moreover fails to disclose many of the essential aspects of the present invention, including the steps of the polycondensation of AB₂ monomers to a low molecular weight polyurea and the immobilization of N-alkylated PE onto the hyperbranched polyurea coating. Hence, US5,908,808 bears no relevance to the present invention.

The method of the present invention finds its use in, among others, a method of manufacturing an implantable or insertable medical device being resistant to microbial growth and/or biofilm formation.

Any device prepared from a biocompatible material having appropriate mechanical properties for the particular application can be used when practicing the present invention. For example, suitable materials include titanium, titanium alloys (such as nickel titanium), nickel alloys, stainless steel, stainless steel alloys, platinum, platinum alloys and mixtures thereof. In other embodiments, suitable materials may include polymeric materials such as polyurethanes, polyamides, polyetheretherketones (PEEK), polyether block amides (PEBA), polytetrafluoroethylene (PTFE), silicones, and mixtures thereof. Polydimethylsiloxane (PDMS) belongs to a group of polymeric organosilicon compounds that are commonly referred to as silicones. PDMS is the most widely used silicon-based organic polymer. PDMS is in general, inert, nontoxic, and non-flammable.

As used herein, the term reactive hydroxyl group refers to any hydroxyl moiety being covalently attached to the surface which can react with a siloxane compound comprising blocked isocyanate groups (hereinafter referred to as "coupling agent"). A surface comprising reactive hydroxyl groups can be provided according to procedures known in the art depending on the substrate used. For example, plasma treatment of PDMS has been described by Whitesides et al. (Langmuir 1991, 7, 1013-1025) to provide PDMS surfaces with hydroxyl groups. Another suitable method to introduce hydroxyl groups on the surface is by using piranha solutions (H₂SO₄/H₂O₂, Kai-Seng Koh, at al, Micromachines 2012, 3, 427-441) Surface hydrophilicity of samples, changes of surface chemistry, surface morphologies of samples, and structural analysis of formed hydroxyapatite can be investigated by contact angle to water, X-ray photoelectron spectrometer (XPS), scanning electron microscopy (SEM), Fourier transform infrared (FTIR) and X-ray diffraction (XRD).

In another embodiment, reactive hydroxyl groups are silanol groups. A silanol is a functional group in silicon chemistry with the connectivity Si-O-H. For example, a silicone rubber substrate like PDMS can be treated with oxygen plasma to alter the surface chemistry, creating silanol (SiOH) groups to the surface using previously described methods. Atmospheric air plasma and argon plasma will work for this application. See for instance Olander et al (Biomacromolecules 2002, 3, 505-510) disclosing argon microwave plasma treatment and subsequent hydrosilylation Alternatively, surfaces can be treated with piranha solutions to obtain hydroxyl groups. See for example Kai-Seng et al. Micromachines 2012, 3, 427-441; doi:10.3390/mi3020427. In step (ii) of a method according to the invention, a siloxane compound comprising blocked isocyanate groups is covalently grafted onto the reactive hydroxyl to serve as coupling agent between the surface and the hyperbranched polyurea coating. Suitable siloxane compounds include 3-isocyanato-propyl trialkoxy silane, 3-mercapto-propyl trialkoxy silane and 3-amino-propyl trialkoxy silane.

In a preferred embodiment, the siloxane coupling agent is of the formula 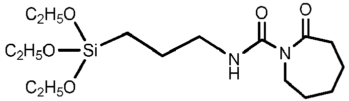 (2-oxo-N(3-triethoxysilyl)propyl)azepane-1-carboxamide). It is readily obtained from γ-amino propyl trialkoxy silane and carbonyl biscaprolactam.

The coupling agent can simply be reacted with the reactive groups by contacting (e.g. by immersion) at least part of the surface with a solution of coupling agent, followed by continued reaction at elevated temperature. For example, an activated surface is immersed for 5-15 minutes in an alcohol solution and thereafter maintained at about 100-120°C for at least 1 hour, preferably at least 2 hours, to allow for covalent binding of coupling agent. Unreacted coupling agent is preferably removed by washing, e.g. in ethanol, under sonication.

As described above, a method of the invention is among others characterized in that it comprises the pre-oligomerization of AB₂ monomers comprising a secondary amine as A-group and blocked isocyanates as B-groups to a low number average molecular weight polyurea of at least 2500 Da.

The polymerization can be done in bulk as well as in a appropriate solvent such as dimethyl formamide (DMF). By heating the system above 125 °C, e.g. 145°C, the polymerization proceeds. The molecular weight depends on the polymerization time. The molecular weight is suitably determined by size exclusion chromatography using methods known in the art, for example as described in Macromol. Chem. Phys. 2012, 213, 1841-1850.

Preferably, oligomerization is performed to obtain polyurea having a Mₙ of at least 3000 Da, more preferably at least 3500 Da, like 4000 Da or even more. The best results coating results are obtained with polyurea oligomers having a molecular weight up to about 5000 Da. In one embodiment, the molecular weight is in the range of 2500 to 5000 Da, preferably 3000- 5000 Da like around 3500, 4000, 4200, 4500 or 4700 Da. Any AB₂ monomer comprising a secondary amine as A-group and blocked isocyanates as flanking B-groups (i.e. forming the structure B-A-B) and capable of forming a hyperbranched structure via a ureido unit can be used to obtain a hyperbranched polyurea coating in a method of the invention.

In a preferred embodiment, the AB₂ monomer is of the general formula 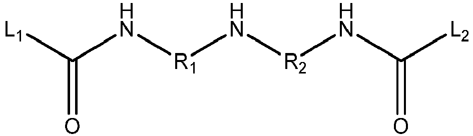 Wherein R₁ and R₂ are aliphatic chains (CH₂)ₘ and (CH₂)ₙ wherein m and n are an integer in the range of 3 to 15, preferably 3 to 8, and wherein. L₁ and L₂ are blocking groups for isocyanates, including but not limiting to caprolactam, phenol, oxime, triazole and malonic esters. Caprolactam is most preferred.
M and n can be the same or they can be different. In one embodiment, m and n are the same. In a specific aspect, m and n are 3, 4, 5 or 6.
AB₂ monomers can be synthesized using methods known in the art. For example, an AB₂ monomer of the general formula herein above is readily obtained by reacting carbonyl biscaprolactam with the appropriate triamine, like bishexamethylene triamine (m and n are 6) or bispentamethylene triamine (m and n are 5).

Following pre-oligomerization, the low molecular weight polyurea is contacted with the surface grafted with coupling agent to covalently anchor the polyurea, and polycondensation is continued by heating, optionally in the presence of AB₂ monomers, to obtain a hyperbranched polyurea coating. Heating is typically performed at a temperature of about 100-150°C, depending on the blocking group L₁ and L₂. for a period of at least 1 hour, preferably at least 1.5 hours. In one embodiment, polymerization is performed at around 145°C for 1-3 hours, preferably under nitrogen. Unreacted compounds can be removed, for example by sequential sonication in ethanol, extraction in DMF overnight, and a second sonication in ethanol.

Depending on the desired thickness of the coating, several rounds of subsequent polycondensation can be performed. For example, a (cross-linked) coating comprising several layers of covalently bound polyurea can be obtained which is robust, reproducible and long-lasting. In one embodiment, step (iv) of a method of the invention comprises at least two, preferably at least three, sequential rounds of continued polycondensation e.g. to obtain a (cross-linked) coating with a thickness of up to several micrometers.

To obtain a coating having antimicrobial properties, an antibacterial functionality is coupled to the hyperbranched coating. More specifically, step (v) of a method of the invention comprises immobilizing onto the hyperbranched polyurea coating a hydrophobic N-alkylated polyamines, such as polyethylenimine (PEI) having antimicrobial properties. N-alkylation comprises alkylation with linear, cyclic or branched C₅-C₁₅ alkyl chains, or aromatic compounds, such as benzyl, preferable method is N-hexylation, N-dodecylation, and N-methylation.

Polyethylenimine based biocides are effective in inhibiting the growth of many microorganisms. As used herein, microorganisms includes single-cell and multi-cell bacteria, fungi, parasites, protozoans, archaea, protests, amoeba, viruses, diatoms, and algae. Microorganisms whose growth may be inhibited by polyethylenimine based biocides of the invention include *Staphylococcus aureus, Staphylococcus epidermidis, Streptococcus faecalis, Bacillus subtilis, Salmonella chloraesius, Salmonella typhosa, Escherichia coli, Mycobacterium tuberculosis, Pseudomonas aeruginosa, Aerobacter aerogenes Saccharomyces cerevisiae, Candida albicans, Aspergillus niger, Aspergillus flares, Aspergillus terreus, Aspergillus verrucaria, Aureobasidium pullulans, Chaetomium globosum, Penicillum funiculosum, Trichophyton interdigital, Pullularia pullulans, Trichoderm sp. madison P-42,* and *Cephaldascus fragans; Chrysophyta, Oscillatoria bometi, Anabaena cylindrical, Selenastrum gracile, Pleurococcus sp., Gonium sp., Volvox sp., Klebsiella pneumoniae, Pseudomonas fluorescens, Proteus mirabilis, Enterobacteriaceae, Acinetobacter spp., Pseudomonas spp., Candida spp., Candida tropicalis, Streptococcus salivarius, Rothia dentocariosa, Micrococcus luteus, Sarcina lutea, Salmonella typhimurium, Serratia marcescens, Candida utilis, Hansenula anomala, Kluyveromyces marxianus, Listeria monocytogenes, Serratia liquefasciens, Micrococcus lysodeikticus, Alicyclobacillus acidoterrestris, MRSA, Bacillus megaterium, Desulfovibrio sulfuricans, Streptococcus mutans, Cobetia marina, Enterobacter aerogenes, Enterobacter cloacae, Proteus vulgaris, Proteus mirabilis, Lactobacillus plantarum, Halomonas pacifica, Ulva linza,* and *Clostridium difficile.* Polyethylenimine based biocides may inhibit the growth of small colonies of microorganisms, as well as biofilms.

There are various ways by which the coating can be provided with N-alkylated PEI. In one embodiment, it comprises tethering PEI to the hyperbranched polyurea coating followed by a two-step N-alkylation of PEI to form quaternary amino groups. This procedure is known per se in the art and typically comprises sequential N-alkylation using halogenated alkanes, for example N-hexylation of PEI with bromohexane followed by N-methylation of N-hexyl-PEI using iodomethane. See for example Lin et al. (Biotechnology and Bioengineering, Vol. 83, 168-172; Behlau et al. (2011, Biomaterials 32(34):8783-8796) or Asri *et al.*

In another embodiment, it comprises N-alkylation of PEI prior to tethering N-alkylated PEI to the hyperbranched polyurea coating. This has the advantage that the substrate surface, e.g. an implant surface, does not need to be exposed to reaction conditions used for alkylation. Prior N-alkylation may comprise the traditional two-step alkylation. In a preferred embodiment, it comprises a one-stage reaction wherein PEI is N-alkylated simultaneously with two different alkyls.

The present inventors observed that triethylamine, commonly used as base in the PEI N-alkylation reaction, causes an undesirable side-reaction with halogenated alkane, e.g. hexylbromide, and moreover yields a coating having a rough surface. It was surprisingly found that these disadvantages can be overcome by preparing N-alkylated PEI by alkylating PEI in the presence of a proton sponge, including 1,6-di-t-butyl pyridine, 1,6-dimethyl pyridine and N,N,N',N'-tetramethylnaphthalene-1,8-diamine (1,8-Bis(dimethylamino)naphthalene, which can react with a proton but not with halogenated alkane.

A method of the invention is advantageously used to modify a substrate having, at least in part, a medical grade polymer surface, preferably selected from the group consisting of plasma-treated silicone rubber, such as plasma-treated medical grade polydimethylsiloxane elastomer (PDMS), polyurethane, polyvinylchloride (PVC).

In another embodiment, the surface is a metal which is biocompatible with the mammalian body, preferably selected from the group consisting of titanium, titanium-alloy, tantalum and tantalum-alloy.

Also provided herein is a substrate coated with an antimicrobial coating obtainable by a method of the invention. Encompassed are continuous coatings covering the entire substrate but also discontinuous local coatings or combinations of local coatings and continuous top coatings. A substrate of the invention is characterized by a strongly adhered antimicrobial coating which is displays a high mechanical strength. A further aspect relates to a method for reducing microbial biofilm formation on the surface of a medical device or implant, comprising the step of providing at least part of the surface of said device or implant with an antimicrobial coating employing a method according to the invention.

The substrate is for example a medical device or implant, for instance a catheter or a prosthesis.In one embodiment, the implant is an orthopedic implant or a cardiovascular implant, for example selected from the group consisting of cardiac valves, alloplastic vessel wall supports, and total artificial heart implants. In another embodiment, the implant is selected from the group consisting of ear tubes, endotracheal tubes, ventilation tubes, cochlear implants and bone anchored hearing devices.
In a specific aspect, the invention provided a coated voice prosthesis. Total laryngectomy, a surgical treatment for extensive cancer of larynx, which alters swallowing and respiration in patients, is typically followed up with a surgical voice restoration procedure comprising tracheoesophageal puncture techniques with insertion of a "voice prosthesis" to improve successful voice rehabilitation. However, microbial or fungal colonization is a major drawback of these devices. Antimicrobials are usually used to prevent the colonization of (silicone rubber) voice prostheses by microorganisms. However, long-term medication induces the development of resistant strains with all associated risks and the development of alternative prophylactic and therapeutic agents, including probiotics and biosurfactants, have been suggested. A voice prosthesis provided with a coating of the present invention is highly effective in inhibiting microbial growth on surfaces of voice prostheses. Herewith, the frequency of replacing voice prostheses in laryngectomized patients can be reduced through microbial biofilm retardation.

In another specific aspect, the substrate is a catheter. The catheter can be a vascular catheter, chronic dwelling gastrointestinal catheter, dialysis catheter, hemodialysis catheter, or chronic dwelling genitourinary catheter. In one embodiment, it is a central venous catheter ("central line", "CVC", "central venous line" or "central venous access catheter"), which is typically placed into a large vein in the neck (internal jugular vein), chest (subclavian vein or axillary vein) or groin (femoral vein). It is used to administer medication or fluids, obtain blood tests, and measure central venous pressure. All catheters can introduce bacteria into the bloodstream, but CVCs are known for occasionally causing *Staphylococcus aureus* and *Staphylococcus epidermidis* sepsis. The problem of central line-associated bloodstream infections (CLABSI) has gained increasing attention in recent years. They cause a great deal of morbidity and deaths, and increase health care costs. The present invention provides unique tools to provide a catheter with an antimicrobial coating, thus reducing the risk of bacterial infections in the bloodstream.

With urinary tract infections as the most frequent implant related hospital acquired infections (HAI) in developed countries, orthopedic implant infections is another major sub-populations within the multifactorial group of HAI (together with infections related to cardiovascular, neurological and gastrointestinal interventions). Infections due to implantation of total hip and total knee prostheses account for about 2% of the HAI, without taking trauma implants into account. Trauma implants or implants for fracture fixation and stabilization, like plates, screws and stabilizing frames, have been described to have an even higher risk for infection, mainly due to the fact that they are used to repair complex injuries and open fractures. Infection together with the eventual loosening of an orthopedic implant explains the limited lifespan of an orthopedic device (generally up to 15 years for an artificial joint).

Since the discovery of antibiotics, (implant) infections have been reduced and implant infections have become less lethal and can even be cured in most cases. However, sometimes the prosthesis has to be removed to allow the patient to recover. Moreover, the extensive use of antibiotics has resulted in an increasing amount of resistant bacterial strains, which makes infections caused by those pathogens challenging. Medical device implantation remains troublesome also in the case of orthopedic implants. Hence, in one embodiment the substrate is an orthopedic implant. Implants in the field of orthopedic and trauma surgery have been used for a long time to restore joint function, reduce pain or stabilize fractures. Both prevention of infection as well as integration of the implant with the host-tissue (mostly bone) are factors of concern. The most frequently used implants for these purposes are made of metallic alloys (for plates and nails for fracture repair and for total joint arthroplasties) and polyethylene (PE) (used in the articulating parts of a prosthesis).

Infection of orthopedic implants and prostheses is a medical issue that has intrigued people since their very first use over two-and-a-half millennia ago. Since that early beginning, lack of fixation and infection has been the major problem with such medical devices. In many cases, it may result in serious discomfort, limb amputation, illness and in many cases it may have even resulted in death of the patient. Thus, postoperative infection associated with implants remains a serious complication in orthopedic surgery. Implant surface treatment according to the present invention is a highly attractive means of reducing the incidence of implant-associated infections. Accordingly, also provided is a method for reducing or eliminating the incidence of an implant-associated infection, comprising implanting a medical device or implant coated with an antimicrobial coating obtainable by according to a method of the invention.

The implant may comprise metallic material, metal oxides, synthetic or natural, cross-linked or non-crosslinked, polymeric materials, ceramics, porcelain, allogenic or xenogenic bone or bone matrix; genetically engineered bone; and combinations thereof. The metallic material is preferably selected from the group consisting of NiTi alloys, stainless steel, titanium or alloys thereof.

Preferably, the polymeric material is non-degradable. For example, the polymeric material is selected from the group consisting of polydimethyl siloxane (PDMS), polypropylene (PP), polyethylene (PE), polyacetylene, cross-linked or non-crosslinked polystyrene, styrene-butadiene rubber, TEFLON®, poly(vinyl chloride) (PVC), polyolefin copolymers, poly(methyl methacrylate), polyphenylene, polyacrylonitrile, carbon fiber, and blends or copolymers thereof.

The coated substrate may comprise at least one therapeutic agent and/or at least one further antimicrobial, e.g. antibacterial, agent. The therapeutic agent can be selected from the group consisting of immunosuppressants, anti-inflammatories, anti-proliferatives, anti-migratory agents, anti-fibrotic agents, cell permeabilizers, proapoptotics, calcium channel blockers, antineoplastics, antibodies, anti-thrombotics, anti-platelet agents, IIb/IIIa blockers, antiviral agents, anti-cancer agents, chemotherapeutics, thrombolytics, vasodilators, antibiotics, growth factor antagonists, free radical scavengers, radiopaque agents, anti-angiogenesis agents, angiogenesis drugs, cyclooxygenase inhibitors, phosphodiesterase inhibitors, cytokine inhibitors, nitrogen oxide donors, cytokine activators, antioxidants, radioactive compounds, steroids, and non-steroidal antiinflammatory drugs and derivatives and analogues thereof. In one embodiment, the coated substrate comprises a cell permeabilizer capable of increasing the permeability of the outer membrane (OM) of a target Gram-negative bacterium such that the activity of the immobilized quaternary ammonium compound is enhanced. Exemplary cell permeabilizers include chelating agents like EDTA, nitrilotriacetic acid (NTA) and succimer (meso-2,3-dimercaptosuccinic acid).

### LEGEND TO THE FIGURES

Figure 1: Microscopic images of glass coating with 5wt% oligomer (20x zoom) according to the invention. Panels A and B show representative sections.
Figure 2: Microscopic images of glass coating with 5wt% monomer (20x zoom) according to the prior art. Panels A and B show representative sections.

### EXPERIMENTAL SECTION

### Materials

Glass slides were purchased from Waldemar Knittel® (Braunschweig, Germany). Polydimethyl siloxane (PDMS) sheets were kindly provided by Atos Medical (Hörby, Sweden). Bishexamethylene triamine, (3-aminopropyl) triethoxysilane, polyethyleneimine (750 kDa, 50 wt% in water), iodomethane, 2-methyl-2-butanol, fluorescein disodium salt, 1-bromohexane and cetyltrimethylammonium chloride were all purchased from Sigma-Aldrich. Potassium hydroxide and dimethylformamide were purchased from Acros organic. Sulfuric acid, hydrogen peroxide and ethanol were obtained from Merck. Methanol and toluene were obtained from Lab-Scan. Carbonyl biscaprolactam (CBC; >99%) was kindly provided by DSM innovation center, ALLINCO® (The Netherlands). All chemicals were used as received.

### Example 1: Synthesis of 2-oxo-N(3-triethoxysilyl)propyl)azepane-1-carboxamide (Coupling agent)

A three neck flask provided with a reflux condenser, a nitrogen inlet and a connector to a vacuum pump, was three times evacuated and flushed with nitrogen, to remove all oxygen. CBC (11.34 g, 45 mmol) and (3-aminopropyl) triethoxysilane (9.95 g, 45 mmol) were dissolved in 40 mL toluene. The reaction was carried out under nitrogen at 80°C overnight. After the solution was cooled down to room temperature toluene was removed under reduced pressure. The obtained coupling agent was stored under nitrogen. The liberated caprolactam was not removed. According to the NMR spectrum, the yield was more than 98%. ¹H-NMR (300 MHz, CDCl₃) δ = 0.60 (2H, m, Si-CH₂), 1.19 (9H, m, Si-O-CH₂-CH₃), 1.65-1.74 (2H , m, Si-CH₂-CH₂ and 4H CO-N-CH₂-(CH₂)₃ (ring)), 2.67 (2H, m, N-CO-CH₂(ring)), 3.28 (2H, m, NH-CH₂), 3.79 (6H, m, Si-O-CH₂), 3.97 (2H, m, CO-N-CH₂, ring), 9.27 (1H, br, NH-CO-N).

### Example 2: Application coupling agent

Glass slides (76 mm x 26 mm) were sonicated for 20 min at RT in dichloromethane. Subsequently, they were provided with silanol groups (Si-OH) by using an oxygen plasma cleaner (Diener electronic, Femto) for 60 seconds under stable vacuum (∼2mTorr).

PDMS sheets were cut into small pieces (2.0 x 1.5 cm) and immersed in toluene for 24 h at room temperature with stirring. Next, the pieces were transferred into hexane and stirred for 24 h at room temperature. The samples were dried overnight at 60°C. The PDMS samples were provided with silanol groups (Si-OH) by using an oxygen plasma cleaner (Diener electronic, Femto) for 60 seconds under stable vacuum (∼2mTorr).

The obtained hydrophilic slides were immersed in the 3v/v% solution of coupling agent (see Example 1) in ethanol for 10 min at RT and then placed in vacuum oven and heated at 110°C for 2 hours under vacuum. The unreacted coupling agent was removed by washing the slides in ethanol for 20 minutes in sonic bath at RT and then dried under nitrogen.

### Example 3: Synthesis of AB₂ monomer

A three necked flask equipped with an inlet and outlet was flushed with nitrogen. Carbonyl biscaprolactam (23.22 g, 92 mmol) and bishexamethylene triamine (9.98 g, 46 mmol) were dissolved in 40 mL toluene and stirred at 80 °C for 20 h in a nitrogen atmosphere. The toluene solution was cooled down to room temperature (RT) and extracted five times with an aqueous solution containing 5 wt% CaCl₂. After the aqueous layer had been removed, the organic layer was concentrated to half its volume using a rotary evaporator. Toluene was removed under reduced pressure and the product was dried in a vacuum oven at 40°C overnight (Yield 80%). ¹H-NMR of AB₂ monomer (400 MHz, CDCl₃): δ 1.33-1.47 (m, 16H, CH₂(CH₂)₄CH₂, 1.70 (m, 12H, CH₂(CH₂)₃CH₂, ring), 2.56 (m, 4H, CH₂NH), 2.67 (m, 4H, NCOCH₂, ring), 3.25 (q, 4H, CONHCH₂), 3.96 (t, 4H, CH₂N, ring), 9.23 (br, 2H, CONH).

### Example 4: Condensation of AB₂ monomers to a low average number molecular weight polyurea

A three-neck flask, provided with a reflux condenser, a nitrogen inlet and a connector to a vacuum pump, was three times evacuated and flushed with nitrogen, to remove all oxygen. To a solution of bishexamethylene triamine (BHTA) (8.81 g, 40.90 mmol) in dry DMF (50 mL) a solution of carbonyl biscaprolactam (20.64 g, 81.81 mmol) in DMF (60 mL) was added and the resulting mixture was stirred at 80 °C overnight under a nitrogen atmosphere. Subsequently, the temperature was raised to 145 °C after which the polymerization started. A number of oligomers with various molecular weights were prepared by heating the monomers during various polymerization times (table 1). The polymer solutions were precipitated in cold water (500 mL). The precipitates were isolated, washed with diethyl ether and dried in the vacuum oven at 40 °C for 48 h. ¹H-NMR (300 MHz, DMSO) δ = 1.07 to 1.57 (8H, m, (CH₂)₄), 1.61 (6H, m (CH₂)₃, ring), 2.65 (2H, m, NCOCH₂), 2.90 to 3.20 (2H, t, CON(CH₂)₂, 2H, m, CONHCH₂), 3.86 (2H, m, CH₂NCO), 6.06 (1H, m, CH₂NHCON(CH₂)₂), 9.13 (1H, t, CONH).

**Table 1: Molecular weights at various residence times**

| Residence time (h) | Mₙ (Da) | M_{w}/Mₙ | BI end-groups |
|---|---|---|---|
| 0.65 | 3,500 | 2.0 | 9 |
| 2 | 4,900 | 2.2 | 12 |
| 4 | 6,700 | 2.3 | 14 |
| 6 | 8,000 | 2.5 | 18 |
| 9 | 9,300 | 3.6 | 25 |
| 10 | 9,500 | 4.1 | 33 |
| 15 | 11,000 | 7.2 | 38 |

### Example 5: Hyperbranched coatings on glass slides

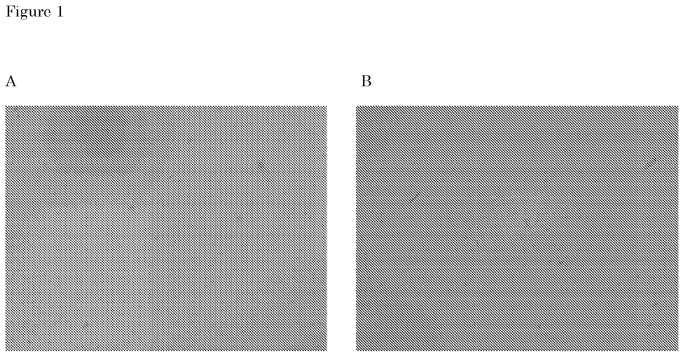

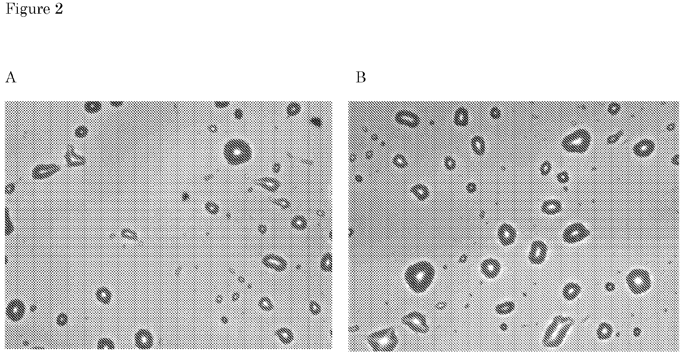

A. 100 pL of a solution of low molecular weight polyurea (Mₙ = 4,900Da; 5 wt% in ethanol) of Example 4 was dropped onto glass slides of Example 2 and spin-coated (2,000 rpm, 60 s). After evaporation of the ethanol the polymerization was continued at 145°C for 2 h under nitrogen. Unreacted compounds were removed by sonication in ethanol for 20 min followed by extraction in DMF at 115°C overnight and sonication again in 100 mL of ethanol for 20 min at RT. The coated glass slides were dried and stored under nitrogen. The microscopic images are shown in Figure 1. The roughness of coated samples was 0.156±0.027 µm, measured by the optical profilometer operating in non-contact mode using white light interferometry (STIL, France). A surface roughness below about 1 µm is generally considered too rough and hence unsuitable for application as medical device. Not only causes a rough surface undesired friction e.g. when inserting it in the body, rougher surfaces also increase the rate of bacterial attachment.
B. As a comparative example, glass slides were coated with AB₂ monomers as has been described in the prior art. Briefly, a solution of AB₂ monomers (5 wt% concentrations in ethanol, 100 pL) was dropped and spin-coated onto glass slides of example 2 (2,000 rpm, 60 s). After evaporation of the ethanol a bulk polymerization was carried out at 145°C for 2 h under nitrogen. Unreacted compounds were removed by sonication in ethanol for 20 min followed by extraction in DMF at 115°C overnight and sonication again in 100 mL of ethanol for 20 min at RT. The coated glass slides were dried and stored under nitrogen. The microscopic images of the coated samples is shown in Figure 2. The roughness was 1.14±0.35 pm, measured by the optical profilometer operating in non-contact mode using white light interferometry (STIL, France).

These data show that pre-oligomerization approach of the present invention resulted in a much lower roughness (0.156±0.027 µm) than a coating obtained via the surface polymerization of monomers (1.14±0.35 µm)

### Example 6: Hyperbranched coatings on PDMS slides

A. 100 pL of a solution of low molecular weight polyurea (Mₙ = 4,900Da; 5 wt% in ethanol) was dropped onto PDMS slides of example 2 and spin-coated (2,000 rpm, 60 s). After evaporation of the ethanol the polymerization was continued at 145°C for 2 h under nitrogen. Unreacted compounds were removed by sonication in ethanol for 20 min followed by extraction in DMF at 115°C overnight and sonication again in 100 mL of ethanol for 20 min at RT. The coated glass slides were dried and stored under nitrogen. The surface topography (roughness) of coated samples was 0.79±0.15 pm, measured by the optical profilometer operating in non-contact mode using white light interferometry (STIL, France).
B. As a comparative example, PDMS slides were also coated with AB₂ monomers.
   100 pL of a solution of the AB₂ monomer (5 wt% in ethanol) was dropped onto PDMS slides and spin-coated (2,000 rpm, 60 s). After evaporation of the ethanol the bulk polymerization was carried out at 145°C for 2 h under nitrogen. Unreacted compounds were removed by sonication in ethanol for 20 min followed by extraction in DMF at 115°C overnight and sonication again in 100 mL of ethanol for 20 min at RT. The coated glass slides were dried and stored under nitrogen. The surface topography (roughness) of coated samples was 2.35±0.32 µm, measured by the optical profilometer operating in non-contact mode using white light interferometry (STIL, France).

### Example 7: Modification of hyperbranched coating with polyethyleneimines

A solution of poly(ethyleneimine) (PEI) in water (50 wt%) was freeze dried overnight (M_{w}=750 kDa) and the residue was dissolved in methanol in 20 wt% concentration. 300 µL of the PEI solution was dropped on coated glass slides of Example 6A and spin casted (2000 rpm, 60 s). The anchoring reactions were carried out at 125°C for 24 h under nitrogen. Unreacted PEI was removed with methanol using ultrasonic bath for 45 min at RT and dried under nitrogen.

### Alkylation of PEI functionalized hyperbranched coating

In a round bottom flask provided with a reflux condenser, coated slides comprising tethered PEI were immersed in 75 mL 1-bromohexane and heated under nitrogen at 90°C overnight. Next 1.07 g of 1,8-bis(dimethylamino)naphthalene (proton sponge) in 25 mL of tert-amyl alcohol was added. The reaction was continued for another 6 hours at 90°C. Afterwards, the coatings were three times sonicated in methanol for 20 min at RT and dried under nitrogen. A second alkylation step was done in a round bottom flask fitted with a reflux condenser. Samples were immersed in a solution of 10 mL iodomethane in 75 mL tert-amyl alcohol. The alkylation reaction was carried out at 42°C overnight; the samples were subsequently sonicated in methanol for 20 min at RT and followed by extraction in methanol at 65°C for 1 day and another sonication in methanol for 20 min at RT. The obtained coatings were dried and stored under nitrogen.

### Example 8: Antibacterial properties of the coated surfaces

### Bacterial culture preparation

Bacteria were grown aerobically overnight at 37°C on a blood agar plate from a frozen stock solution. Plates were kept at 4°C and used no longer than for 2 weeks. A single bacterial colony from a blood agar plate was inoculated in 10 mL trypton soya broth (TSB, OXOID, England) in case of Gram-positive bacteria or 10 mL Todd Hewitt broth (THB, OXOID, England) in case of Gram-negative bacteria, and incubated at 37°C for 24 h. This pre-culture was used to inoculate 200 mL of TSB or THB and incubated for 16 h. Bacteria were harvested by centrifugation at 5000 × *g* for 5 min and washed twice with phosphate buffered saline (PBS buffer, 10 mM potassium phosphate, 150 mM NaCl, pH 7). Bacteria were sonicated on ice for 3 × 10 s at 30 W (Vibra Cell model 375, Sonics and Materials, USA) to break up aggregates if needed. This established procedure did not cause cell lysis.

### Petrifilm assay

The Petrifilm assay (Petrifilm™ Aerobic Countplate, 3M, USA) was used to determine killing bacteria on contact under nutrient rich conditions. Bacterial concentrations of 10⁶, 10⁵ and 10⁴ per mL in PBS buffer were used. The bottom of the Petrifilm containing gelling agent was first swelled with 1 mL sterile demi water for 30 min. Next, 10 pL of bacterial suspensions at the appropriate concentration was dropped on coated and un-coated samples that were placed on the bottom film of the Petrifilm. After the top-lid was placed on the coated samples of example 6 the Petrifilms were incubated at 37°C for 48h. Bacterial suspension without a sample in between the films (10 µL) was used as a control. After the incubation time numbers of the CFU per cm² were counted. The results with various bacterial strains is given in Table 2.

**Table 2. Antibacterial properties of coated substrate against various bacterial strains and at various concentrations.**

| Bacterial strain | Control* 10² | 10² | 10³ | 10⁴ | 10⁵ |
|---|---|---|---|---|---|
| *Staphylococcus epidermidis* ATCC12228 | 74±5 | 0 | 0 | 0 | 0 |
| *Staphylococcus epidermidis* 3081 | 58±9 | 0 | 0 | 3±1 | - |
| *Escherichia coli* ATCC 1110 | 80±7 | 0 | 6±2 | 43±4 | - |
| *Escherichia coli* Hu734 | 77±4 | 12±7 | 140±16 | - | - |

| | | | | | |
|---|---|---|---|---|---|
| *In the control experiment a calculated number of 100 bacteria were suspended on the substrate to check the viability. | | | | | |

These data show that the antibacterial coating of example 6 kills >99% of the *Staphylococcus epidermidis* ATCC 12228, *Staphylococcus epidermidis* 3081, *Escherichia coli* ATCC 1110 and about 85% of *Escherichia coli* Hu734.

### Example 9: Bactericidal properties against Gram negative bacteria in the presence of EDTA.

Coated PDMS slides described in example 7 (PDMS-PEI⁺) were evaluated according the procedure of example 8 for their bactericidal effect against Gram negative bacteria (strains ATCC 15597 and Hu 734), in the absence or presence of EDTA. The results (Table 3) show that a permeabilisor, such as EDTA, has a profound influence on the antibacterial properties of said coatings against Gram negative bacteria. EDTA as such has no antibacterial properties at all.

**Table 3**

| **Sample** | **Bacterial challenge per sample (5 cm²)** | | |
|---|---|---|---|
| | **10²** | **10³** | **10⁴** |
| *E.coli* ATCC 15597 (triplicates) | | | |
| **Control** (viability check) | 79 ± 3 | - | - |
| **PDMS uncoated** | 65 ± 6 | TMTC | TMTC |
| **PDMS-PEI⁺** | 6 ± 3 | 65 ± 8 | TMTC |
| **control + 0.1 mM EDTA** | 76 ± 4 | - | - |
| **control + 1 mM EDTA** | 78 ± 2.5 | - | - |
| **PDMS-PEI⁺ + 0.1 mM EDTA** | 0 | 3 ± 1.5 | 41 ± 4.5 |
| **PDMS-PEI⁺ + 1 mM EDTA** | 0 | 0 | 25 ± 6 |

| ***E.** coli* **Hu 734 (triplicates)** | | | |
|---|---|---|---|
| **Control** (viability check) | 78 ± 4 | - | - |
| **PDMS uncoated** | 73 ± 5 | TMTC | TMTC |
| **PDMS-PEI⁺** | 12 ± 7 | 140 ± 16 | TMTC |
| **control + 0.1 mM EDTA** | 76 ± 8 | - | - |
| **control + 1 mM EDTA** | 75 ± 6 | - | - |
| **PDMS-PEI⁺ + 0.1 mM EDTA** | 2 ± 1.5 | 12 ± 4 | 38 ± 8 |
| **PDMS-PEI⁺ + 1 mM EDTA** | 0 | 4 ± 2.5 | 26 ± 6.5 |

## Claims

1. A method for providing a substrate with an antimicrobial coating by immobilizing a quaternary ammonium compound onto the surface of said substrate, comprising the steps of :
(i) providing a surface comprising reactive hydroxyl groups;
(ii) covalently grafting onto said reactive hydroxyl groups a siloxane coupling agent comprising a blocked isocyanate group;
(iii) polycondensation of AB₂ monomers comprising a secondary amine as A-group and blocked isocyanates as B-groups to obtain a low number average molecular weight (Mₙ) polyurea of at least 2500 Da;
(iv) contacting said low molecular weight polyurea with the surface grafted with coupling agent to covalently anchor the polyurea, and continuing polycondensation by heating, optionally in the presence of AB₂ monomers, to obtain a hyperbranched polyurea coating; followed by
(v) immobilizing onto said hyperbranched polyurea coating a hydrophobic N-alkylated polyethylenimine (PEI) having antimicrobial properties.

2. Method according to claim 1, wherein said coupling agent is (2-oxo-N(3-triethoxysilyl)propyl)azepane-1-carboxamide.

3. Method according to claim 1 or 2, wherein said AB₂ monomers are of the general formula 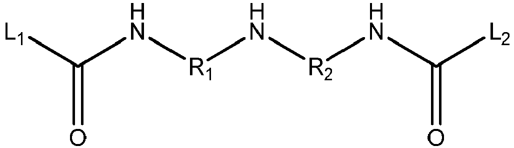 wherein
R₁ and R₂ are aliphatic chains (CH₂)ₘ and (CH₂)ₙ wherein m and n are an integer in the range of 3 to 15, preferably 3 to 8, and
L₁ and L₂ are blocking groups, preferably selected from caprolactam, phenol, oxime, triazole and malonic esters.

4. Method according to any one of the preceding claims, wherein step (iii) comprises polycondensation of AB₂ monomers to a number average molecular weight in the range of 2500 to 5000 Da, preferably 3000- 5000 Da.

5. Method according to any one of the preceding claims, wherein step (v) comprises preparing N-alkylated PEI by alkylating PEI in the presence of 1,8-Bis(dimethylamino)naphthalene, 1,6-di-t-butyl pyridine or 1,6-dimethyl pyridine .

6. Method according to any one of the preceding claims, wherein step (v) comprises immobilizing PEI to the hyperbranched polyurea coating followed by N-alkylation of PEI.

7. Method according to any one of claims 1-5, wherein step (v) comprises N-alkylation of PEI prior to immobilizing N-alkylated PEI to the hyperbranched polyurea coating.

8. Method according to any one of the preceding claims, wherein N-alkylation comprises alkylation with a linear or branched C₅-C₁₅ alkyl chain, preferably N-hexylation or N-dodecylation, and N-methylation.

9. Method according to any one of the preceding claims, wherein N-alkylation comprises a two-stage alkylation of PEI, preferably N-hexylation followed by N-methylation of N-hexyl-PEI.

10. Method according to any one of claims 1-8, wherein N-alkylation comprises a one-stage alkylation of PEI.

11. Method according to any one of the preceding claims, wherein said surface is a medical grade polymer, preferably selected from the group consisting of plasma-treated silicone rubber, such as plasma-treated medical grade polydimethylsiloxane elastomer (PDMS), polyurethane, and polyvinylchloride (PVC).

12. Method according to any one of claims 1 to 10, wherein said surface is a metal which is biocompatible with the mammalian body, preferably selected from the group consisting of titanium, titanium-alloy, tantalum and tantalum-alloy.

13. Method according to any one of the preceding claims wherein substrate is a medical device or implant.

14. Method according to claim 13, wherein the substrate is selected from the group consisting of a catheter, a prosthesis, an orthopedic implant and a cardiovascular implant.

15. A substrate coated with an antimicrobial coating, obtainable by a method according to any one of claims 1-14.

16. Coated substrate according to claim 15, comprising at least one therapeutic agent and/or a further antimicrobial agent.

17. Coated substrate according to claim 15 or 16, being a medical device or implant, preferably selected from the group consisting of a catheter, a prosthesis, an orthopedic implant and a cardiovascular implant.

18. A method for reducing microbial biofilm formation on the surface of a substrate, preferably a medical device or implant, comprising the step of providing at least part of the surface of said substrate with an antimicrobial coating obtainable by a method according to any one of claims 1-14.

19. A method for reducing or eliminating the incidence of an implant-associated infection, comprising implanting a coated medical device or implant according to claim 17 in a subject in need thereof.

## Patentansprüche

1. Verfahren zum Bereitstellen eines Substrats mit einer antimikrobiellen Beschichtung durch Immobilisieren einer quaternären Ammoniumverbindung auf der Oberfläche des Substrats, umfassend die Schritte von:
(i) Bereitstellen einer Oberfläche, umfassend reaktive Hydroxylgruppen;
(ii) kovalentes Aufpfropfen auf die reaktiven Hydroxylgruppen eines Siloxankupplungsmittels, umfassend eine blockierte Isocyanatgruppe;
(iii) Polykondensation von AB₂-Monomeren, umfassend ein sekundäres Amin als A-Gruppe und blockierte Isocyanate als B-Gruppen, um einen Polyharnstoff mit niedrigem zahlendurchschnittlichen Molekulargewicht (Mₙ) von wenigstens 2500 Da zu erhalten;
(iv) Inkontaktbringen des Polyharnstoffs mit niedrigem Molekulargewicht mit der Oberfläche, aufgepfropft mit Haftvermittler, um den Polyharnstoff kovalent zu verankern, und Fortsetzen von Polykondensation durch Erhitzen, optional in der Gegenwart von AB₂-Monomeren, um eine hyperverzweigte Polyharnstoffbeschichtung zu erhalten; gefolgt von
(v) Immobilisieren auf der hyperverzweigten Polyharnstoffbeschichtung eines hydrophoben N-alkylierten Polyethylenimins (PEI) mit antimikrobiellen Eigenschaften.

2. Verfahren nach Anspruch 1, wobei der Haftvermittler (2-Oxo-N(3-triethoxysilyl)propyl)azepan-1-carboxamid ist.

3. Verfahren nach Anspruch 1 oder 2, wobei die AB₂-Monomere von der allgemeinen Formel 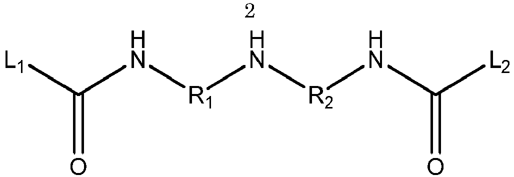 sind, wobei
R₁ und R₂ aliphatische Ketten (CH₂)ₘ und (CH₂)ₙ sind, wobei m und n eine ganze Zahl in dem Bereich von 3 bis 15, vorzugsweise 3 bis 8 sind, und
L₁ und L₂ blockierende Gruppen sind, vorzugsweise ausgewählt aus Caprolactam, Phenol, Oxim, Triazol und Malonsäureestern.

4. Verfahren nach einem der vorhergehenden Ansprüche, wobei Schritt (iii) Polykondensation von AB₂-Monomeren bis zu einem zahlendurchschnittlichen Molekulargewicht in dem Bereich von 2500 bis 5000 Da, vorzugsweise 3000-5000 Da umfasst.

5. Verfahren nach einem der vorhergehenden Ansprüche, wobei Schritt (v) Herstellen von N-alkyliertem PEI durch Alkylieren von PEI in der Gegenwart von 1,8-Bis(dimethylamino)naphthalin, 1,6-Di-t-butylpyridin oder 1,6-Dimethylpyridin umfasst.

6. Verfahren nach einem der vorhergehenden Ansprüche, wobei Schritt (v) Immobilisieren von PEI an der hyperverzweigten Polyharnstoffbeschichtung, gefolgt von N-Alkylierung von PEI, umfasst.

7. Verfahren nach einem der Ansprüche 1 - 5, wobei Schritt (v) N-Alkylierung von PEI vor Immobilisierung von N-alkyliertem PEI an der hyperverzweigten Polyharnstoffbeschichtung umfasst.

8. Verfahren nach einem der vorhergehenden Ansprüche, wobei N-Alkylierung Alkylierung mit einer linearen oder verzweigten C₅-C₁₅-Alkylkette, vorzugsweise N-Hexylierung oder N-Dodecylierung, und N-Methylierung, umfasst.

9. Verfahren nach einem der vorhergehenden Ansprüche, wobei die N-Alkylierung eine zweistufige Alkylierung von PEI, vorzugsweise N-Hexylierung, gefolgt von N-Methylierung von N-Hexyl-PEI, umfasst.

10. Verfahren nach einem der Ansprüche 1 - 8, wobei N-Alkylierung eine einstufige Alkylierung von PEI umfasst.

11. Verfahren nach einem der vorhergehenden Ansprüche, wobei die Oberfläche ein Polymer von medizinischer Qualität ist, vorzugsweise ausgewählt aus der Gruppe bestehend aus plasmabehandeltem Silikonkautschuk, wie plasmabehandeltem Polydimethylsiloxanelastomer (PDMS) von medizinischer Qualität, Polyurethan, und Polyvinylchlorid (PVC).

12. Verfahren nach einem der Ansprüche 1 bis 10, wobei die Oberfläche ein Metall ist, das mit dem Säugetierkörper biokompatibel ist, vorzugsweise ausgewählt aus der Gruppe bestehend aus Titan, Titanlegierung, Tantal und Tantallegierung.

13. Verfahren nach einem der vorhergehenden Ansprüche, wobei das Substrat eine medizinische Vorrichtung oder ein Implantat ist.

14. Verfahren nach Anspruch 13, wobei das Substrat ausgewählt ist aus der Gruppe, bestehend aus einem Katheter, einer Prothese, einem orthopädischen Implantat und einem kardiovaskulären Implantat.

15. Substrat, beschichtet mit einer antimikrobiellen Beschichtung, erhältlich durch ein Verfahren nach einem der Ansprüche 1 - 14.

16. Beschichtetes Substrat nach Anspruch 15, umfassend wenigstens ein therapeutisches Mittel und/oder ein weiteres antimikrobielles Mittel.

17. Beschichtetes Substrat nach Anspruch 15 oder 16, wobei es eine medizinische Vorrichtung oder ein Implantat ist, vorzugsweise ausgewählt aus der Gruppe bestehend aus einem Katheter, einer Prothese, einem orthopädischen Implantat und einem kardiovaskulären Implantat.

18. Verfahren zum Reduzieren mikrobieller Biofilmbildung auf der Oberfläche eines Substrats, vorzugsweise einer medizinischen Vorrichtung oder eines Implantats, umfassend den Schritt des Bereitstellens wenigstens eines Teils der Oberfläche des Substrats mit einer antimikrobiellen Beschichtung, erhältlich durch ein Verfahren nach einem der Ansprüche 1 - 14.

19. Verfahren zum Reduzieren oder Eliminieren des Auftretens einer implantatassoziierten Infektion, umfassend Implantieren einer beschichteten medizinischen Vorrichtung oder eines Implantats nach Anspruch 17 in einen Patienten, der dies benötigt.

## Revendications

1. Procédé de fourniture d'un substrat avec un revêtement antimicrobien par immobilisation d'un composé d'ammonium quaternaire sur la surface dudit substrat, comprenant les étapes de :
(i) fourniture d'une surface comprenant des groupes hydroxyle réactifs ;
(ii) greffage covalent sur lesdits groupes hydroxyle réactifs d'un agent de couplage de siloxane comprenant un groupe isocyanate bloqué ;
(iii) polycondensation de monomères AB₂ comprenant une amine secondaire comme groupe A et des isocyanates bloqués comme groupes B afin d'obtenir une polyurée de faible masse moléculaire moyenne en nombre (Mₙ) d'au moins 2 500 Da ;
(iv) contact de ladite polyurée de faible masse moléculaire avec la surface greffée avec un agent de couplage pour fixer de manière covalente la polyurée, et continuation de polycondensation par chauffage, éventuellement en présence de monomères AB₂, pour obtenir un revêtement de polyurée hyperramifiée ; suivi par
(v) l'immobilisation sur ledit revêtement de polyurée hyperramifiée d'une polyéthylèneimine N-alkylée hydrophobe (PEI) présentant des propriétés antimicrobiennes.

2. Procédé selon la revendication 1, dans lequel ledit agent de couplage est le (2-oxo-N(3-triéthoxysilyl)propyl)azépane-1-carboxamide.

3. Procédé selon la revendication 1 ou 2, dans lequel lesdits monomères AB₂ sont de la formule générale 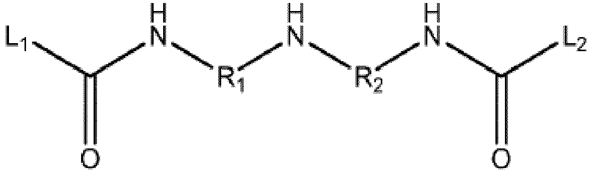 dans laquelle
R₁ et R₂ sont des chaînes aliphatiques (CH₂)ₘ et (CH₂)ₙ, dans lesquelles m et n sont un nombre entier dans l'intervalle de 3 à 15, de préférence de 3 à 8, et
L₁ et L₂ sont des groupes de blocage, de préférence choisis parmi le caprolactame, le phénol, un oxime, un triazole et des esters maloniques.

4. Procédé selon l'une quelconque des revendications précédentes, dans lequel l'étape (iii) comprend la polycondensation de monomères AB₂ à une masse moléculaire moyenne en nombre dans l'intervalle de 2 500 à 5 000 Da, de préférence 3 000-5 000 Da.

5. Procédé selon l'une quelconque des revendications précédentes, dans lequel l'étape (v) comprend la préparation de PEI N-alkylée par alkylation de PEI en présence de 1,8-bis(diméthylamino)naphtalène, 1,6-di-t-butylpyridine ou 1,6-diméthyl-pyridine.

6. Procédé selon l'une quelconque des revendications précédentes, dans lequel l'étape (v) comprend l'immobilisation de PEI sur le revêtement de polyurée hyperramifiée suivie par une N-alkylation de PEI.

7. Procédé selon l'une quelconque des revendications 1-5, dans lequel l'étape (v) comprend la N-alkylation de PEI avant l'immobilisation de PEI N-alkylée sur le revêtement de polyurée hyperramifiée.

8. Procédé selon l'une quelconque des revendications précédentes, dans lequel la N-alkylation comprend l'alkylation avec une chaîne alkyle en C₅-C₁₅ linéaire ou ramifiée, de préférence une N-hexylation ou N-dodécylation, et N-méthylation.

9. Procédé selon l'une quelconque des revendications précédentes, dans lequel la N-alkylation comprend une alkylation en deux étapes de PEI, de préférence une N-hexylation suivie par une N-méthylation de N-hexyl-PEI.

10. Procédé selon l'une quelconque des revendications 1-8, dans lequel la N-alkylation comprend une alkylation en une étape de PEI.

11. Procédé selon l'une quelconque des revendications précédentes, dans lequel ladite surface est un polymère de qualité médicale, de préférence choisi dans le groupe consistant en caoutchouc de silicone traité au plasma, tel qu'un élastomère de polydiméthylsiloxane de qualité médicale traité au plasma (PDMS), polyuréthane, et poly(chlorure de vinyle) (PVC).

12. Procédé selon l'une quelconque des revendications 1 à 10, dans lequel ladite surface est un métal qui est biocompatible avec le corps d'un mammifère, de préférence choisi dans le groupe consistant en titane, alliage de titane, tantale et alliage de tantale.

13. Procédé selon l'une quelconque des revendications précédentes, dans lequel le substrat est un dispositif ou implant médical.

14. Procédé selon la revendication 13, dans lequel le substrat est choisi dans le groupe consistant en un cathéter, une prothèse, un implant orthopédique et un implant vasculaire.

15. Substrat revêtu d'un revêtement antimicrobien, pouvant être obtenu par un procédé selon l'une quelconque des revendications 1-14.

16. Substrat revêtu selon la revendication 15, comprenant au moins un agent thérapeutique et/ou un autre agent antimicrobien.

17. Substrat revêtu selon la revendication 15 ou 16, étant un dispositif ou implant médical, de préférence choisi dans le groupe consistant en un cathéter, une prothèse, un implant orthopédique et un implant cardiovasculaire.

18. Procédé de réduction de formation de biofilm microbien sur la surface d'un substrat, de préférence un dispositif ou implant médical, comprenant l'étape de fourniture d'au moins une partie de la surface dudit substrat avec un revêtement antimicrobien pouvant être obtenu par un procédé selon l'une quelconque des revendications 1-14.

19. Procédé de réduction ou d'élimination de l'incidence d'une infection associée à un implant, comprenant l'implantation d'un dispositif ou implant médical revêtu selon la revendication 17 chez un sujet en besoin de celle-ci.
